# EUROPEAN PATENT APPLICATION

(11) **EP 4 748 240 A1**
(43) Date of publication of application: **27.05.2026**
(21) Application number: 24214226.3
(22) Date of filing: 20.11.2024
(51) Int. Cl.: A23L 33/19, A23J 1/20, A23J 3/08, A23L 33/00

(54) **FOOD COMPOSITIONS CONTAINING HEAT TREATED MILK FILTRATE PROTEINS**

(71) Applicant: HHNW Singapore Pte. Limited, 048619 Singapore (SG)
(72) Inventor: Lane, Jonathan, Fermoy, Co Cork, P61K202 (IE); Kondrashina, Alina, Fermoy, Co Cork, P61K202 (IE); Walsh, Clodagh, Fermoy, Co Cork, P61K202 (IE); O'Hea, Reuben, Fermoy, Co Cork, P61K202 (IE); Bedi, Akash, Fermoy, Co Cork, P61K202 (IE)
(74) Representative: Straus, Alexander

(57) **Abstract**

The present invention provides food compositions containing whey proteins, the structure of which had been essentially conserved during the preparation process. The invention further provides the use of such food compositions for improving the health status of a subject.

## Description

### FIELD OF THE INVENTION

The present invention pertains to the field of food compositions containing whey proteins, in particular infant milk formulas. The present invention further relates to the use of milk proteins obtained via microfiltration and containing whey proteins for improving the intestinal health, immune status and eventually growth of a subject. The invention is also directed to a method for formulating and processing food compositions, such as dairy powder, containing milk proteins obtained via microfiltration and containing whey proteins, wherein the structure and functionality of the whey proteins in the final product are essentially conserved.

### BACKGROUND

Whey proteins are an important fraction of mammal milk. While in cow's milk the share of whey proteins is second to that of casein, whey proteins are dominant in human breast milk providing the newborn with a source for amino acids, especially those the human body cannot synthesize on its own. Whey proteins also comprise immunoglobulins assisting the neonates not yet fully developed immune system and supporting maturation of the intestinal barrier. Among others for these reasons' whey proteins are considered as a valuable and high-grade nutritional component being presently used in muscle build-up and in particular as a component for infant formulas in an attempt to more closely resemble mother's milk.

Since milk, and processed milk streams such as whey, is derived from a natural, biological source, it inherently contains micro-organisms, which need to be removed prior to being able to provide the same as a nutritional component to mammals. The known milk processing and separation techniques, such as cheese production, may incur additional conta'minations, resulting in a microbial load being not satisfactory for human and particularly infant consumption. In order to effectively kill present bacteria, whole milk, skimmed milk, casein and whey enriched side streams are subjected to a pasteurisation step, i.e. a heat treatment at high temperature such as from about 85°C to about 95 °C for about 30 - 90 s.

Yet, whey proteins are known to be quite temperature sensitive resulting in the proteins undergoing structural changes, in the generation of advanced glycation end-products and eventual loss of functionality including health promoting function. For this reason, manufacturing products with whey proteins, that conserve their functionality is still a challenge for the food industry. In order to produce whey proteins in a native state, novel separation techniques were introduced, such as e.g. ultrafiltration, microfiltration and other types of filtration techniques. They allow upon filtering skim milk to retain larger proteins, such as casein, while permeating whey proteins with a reduced bacterial load. Thereby additional heat treatment steps may not always be required as per the standard cheese whey production process.

When combining isolated whey proteins with other ingredients to provide desired food compositions an additional heat treatment is required to ensure a minimum bacterial load in the final product. At present such heat treatment is again carried out at temperatures and a duration as above, which is known to reduce the bioactivity of proteins included in the food composition.

In view of the problems of the prior art there is a need in the art to provide whey proteins containing food compositions, wherein the native protein's bioactivity essentially remains preserved. Another problem resides in providing methods for preparing such products.

### SUMMARY OF THE INVENTION

In order to solve the above-mentioned problems of the prior art, the present invention been accomplished.

Thus, in one aspect the present invention provides whey proteins containing food compositions, wherein the whey proteins included in the compositions have been isolated via a micro-filtration technique and wherein the composition had been treated at a temperature of from about 71.5 to about 82 °C for a time period of from about 10 to about 60 seconds (s). The food compositions thus prepared contain the milk proteins isolated from milk via a micro-filtration technique and comprising whey proteins in an essentially native state (native whey proteins).

The food composition may contain the native whey proteins in an amount of 1 - 80 % and may contain any of additional ingredients conventionally used in industry for preparing food compositions, such as e.g. excipients, nutrients, minerals, vitamins, oligosaccharides, digestible carbohydrates, amino acids or, milk fat and/or a combination thereof.

The present food compositions can be provided in any form desired, i.e. in liquid or solid form and depending on the additional ingredients may have the form of an infant formula, a nutritional product for children, bulk dairy powder, formulated adult food, a food additive, a food supplement, a pet companion food or an animal feed.

The food compositions of the present invention are useful for enhancing a healthy growth and development of a subject, for enhancing its immune status and for supporting the intestinal barrier integrity, maturation and/or recovery after the damage in a subject.

The present food compositions may be prepared by a method comprising providing native whey proteins via a microfiltration, combining said whey proteins with other ingredients for a desired food compositions and subjecting the composition to a heat treatment at a temperature of from about 71.5 - 82 °C and for a duration of from 10 - 60 s.

### IN THE FIGURES

Fig. 1 is a diagram schematically showing preparation of Infant Milk Formulations (IMF) with whey proteins obtained via the conventional way during cheese production and via microfiltration and subjecting them to different temperatures. T1-T3 are samples containing standard whey proteins solely, T4-T6 are samples containing whey proteins obtained via microfiltration. T1 - standard whey pasteurised within IMF at 75.5°C, T2 - standard whey at 85°C, T3 - standard whey at 95°C, and T4 -native whey at 75.5°C; T5 native whey at 85°C; T6 - native whey at 95°C.
Fig. 2 is a diagram showing the solubility index of the infant milk formulas with following pasteurisation temperatures: T1 and T4 at 75.5°C; T2 and T5 at 85°C; and T3 and T6 at 95°C. T1-T3 are samples containing standard whey proteins solely, T4-T6 are samples containing native whey proteins solely.
Fig. 3 is a diagram showing the content of IgG (A) and lactoferrin (B) in the infant milk formulas of Fig. 1, measured by ELISA, n=4 (biological duplicate in technical duplicate).
Fig. 4 is a diagram showing transepithelial electrical resistance (TEER), expressed as %, where 100% is TEER at time 0 for each well, measured in Caco-2 monolayers after 4h incubation with wet mix IMFs, n=4 (biological replicates).
Fig. 5 is a diagram showing immunity modulation as determined by expression of IL-8.

### DETAILED DESCRIPTION OF THE INVENTION

In the extensive studies leading to the present invention, commercial cheese whey proteins products and native whey proteins produced via a membrane filtration process were used to produce stage I infant milk formulations. These formulations were subjected to various pasteurisation temperatures at different time lengths to analyse the effect of the total heat load on the protein state and functionality. The formulations were examined on their composition status, their digestibility and functionality and it was surprisingly found that when subjecting formulations containing native whey proteins to a temperature of from 71.5 - 82 °C for a time period of from 10 - 60 s, the microbial load could successfully be minimized, while the bioactive molecules contained in the native whey proteins still preserved their structure and functionality, in contrast to using whey proteins that had not been isolated via filtration methods, e.g. been isolated from side-streams and subjected before to conventional pasteurisation temperatures of 85-95 °C.

Without wishing to be bound by any theory it is presently contemplated that the surprising results observed may be due to a different reaction behaviour of the whey's proteins isolated via microfiltration vs. whey' proteins isolated via conventional ways. In principle heat treatment of proteins leads to a partial denaturation thereof, which denaturation status may to some extent be reversed with time, but also lead to breakage and formation of additional terminal ends. Apart from that, when proteins are exposed to higher temperatures in the presence of carbohydrates, the proteins may undergo a Maillard reaction, i.e. a reaction of terminal amino acids in the proteins with sugars that leads to the development of undesired Amadori products. The linkage of sugars to the proteins is assumed to prevent proper refolding. Whey obtained the conventional way contains an amount of lactic sugars, which is not present to the same level, when whey proteins are isolated via microfiltration. As a result whey proteins obtained via conventional routes and heat treated for further processing do already contain Amadori products and more terminal ends than the native proteins.

In contrast, whey proteins isolated via microfiltration are subjected to higher temperatures first when processing the same with the final product. Here both, the heat treated whey proteins obtained via conventional ways or the whey proteins obtained via microfiltration are exposed to heat in the presence of carbohydrates to be included in the final product to be produced. Since the heat treated whey proteins do obviously already contain more terminal end due to their pre-treatment, they may form more Amadori-products as native whey proteins, which more Amadori products add to those already present. This obviously adds to the lesser renaturation capability of conventional, heat treated whey proteins as observed in the present invention.

Apart from the above consideration also a higher concentration of essential amino acids, such as lysine, methionine, isoleucine, leucine, phenylalanine, threonine, tryptophane and valine and also histidine, an amino acid specifically important for babies, could be observed in a whey protein isolate obtained via microfiltration as compared to a whey isolate obtained in a conventional way. Likewise, a higher concentration of conditional essential amino acid for infant and children, namely arginine, glutamine, glycine, proline, taurine, cysteine and tyrosine could be observed, as compared to a conventional whey isolate.

The present invention thus provides a native whey proteins-containing food composition, wherein the native whey proteins included essentially exhibits a bioactivity as the milk used as starting material. This is achieved by providing native whey proteins obtained from milk via a filtration technique and mixing the same with the other food ingredients used for preparing a desired food composition.

The stream used as starting material may be milk microfiltration permeate. The terms "native whey proteins" as used herein shall designate whey proteins obtained directly from milk or skim milk, which had not been subjected to a harsh pre-treatment so that the whey proteins essentially maintain their structure and functionality as in the original source, i.e. milk.

The whey proteins may be isolated by a filtration technique, allowing separation of whey proteins from other milk components and bacteria without disturbing their native state. Non limiting examples for such filtration techniques are e.g. ultrafiltration, or microfiltration or occasionally combined with nanofiltration or ion exchange methods. These filtration techniques may be carried out at ambient or preferably lower temperatures, in particular at temperatures in a range of from about 4 to about 15 °C, preferably from about 6 to about 10 °C. If desired the native whey proteins obtained in this way may be subjected to additional processing steps e.g. for removing other small size milk components, such as lactose etc., to increase digestibility and absorption by the body, or for increasing the level of bioactive peptides therein.

The native whey proteins thus obtained are then mixed with other ingredients of a food composition to be prepared, in general, lipids, carbohydrates, minerals, vitamins, other nutrients via conventional techniques, such as wet-mixing, dry-blending, etc. In general, the native whey proteins are mixed with further ingredients in an amount desired for a given food composition and may generally range of from about 1 to about 80 %, preferably from about 4 to about 50 %, all related to the weight of the final food composition. Ranges of from 5-10 wt.-%, preferably 5 - 8 wt.-% more preferably 6 - 7 wt.-% may be preferred. Especially for infant formulas the amount may be selected to range of from about 1 to about 7 wt-.%, depending on the stages of the infant formulas, e.g. for an stage I infant formula of from about 6 to about 7 wt.-% for stages II and III from about 1 to about 6 wt.- %, preferably from about 1 to about 3 wt.-%.

Subsequently the thus mixed composition is subjected to a mild pasteurisation step at a temperature ranging from about 71.5 to about 82 °C for a time period of from about 10 to about 60 s.

According to preferred embodiments the temperature range is from about 72,5 C or 73.5 °C to about 82 °C, about 73.5 °C to about 81 °C, about 73.5 to about 80 °C or about 79 °C or about 78 °C, about 75.5 to about 81 °C, more preferably about 75.5 to about 80 °C, or about 75.5 to about 79 °C, or about 75.5 to about 78 °C, or about 75.5 to about 77 °C. It is understood that any of the temperatures 82 °C, 81 °C, 80 °C, 79 °C, 78 °C, 77 °C, 76 °C, 75,5 °C, 73,5 °C, 72,5 °C or 71,5 °C represents an embodiment of the invention.

In principle, the time period for exposing the whey proteins to the respective temperature as above needs to be sufficient to ensure a full exposure of the mixed composition for achieving complete inactivation of pathogens, but likewise maintaining the functionality of the whey proteins and is in general to be selected from about 10 to about 60 s. According to preferred embodiments the time period ranges of from about 10 to about 50 s, or from about 10 to about 40 s, or from about 20 to about 50 s, or from about 20 to about 60 s, or from about 20 to about 50 s, or from about 20 to about 30 s, or from about 15 to about 50 s, or from about 15 to about 40 s or from about 15 to about 30 s, with a duration of from about 20 to about 30 being most preferred. It is to be understood that any range between said 10 - 60 s shall be considered to explicitly be disclosed and comprised as are the individual temperatures within said range, i.e. 10 s, 11s, 12s, 13s, 14s, 15s, etc. 20s, 21s, 22s, 23s, 24s 25, etc., 30s, 31s, 32s, 33s, 34s, 35s etc. 41s, 42s, 43s, 44s, 45, etc. etc.

The native whey proteins included in a food composition and pasteurised in such a way preserves the beneficial properties of whey proteins.

Apart from the native whey proteins the food product according to the invention may also contain additional ingredients as used in the art. Examples for such ingredients are excipients, e.g. lipids, carbohydrates, enzymes, other nutrients, minerals, and/or vitamins, which may be derived from the food composition itself or which may be added. Also, other bio-functional components may be included or be inherently present in the food compositions, such as, specific amino acids, preferably essential amino acids, pre-biotics or (oligo-)-saccharides, such as e.g. 2'-fucosyllactose (2'-FL), 3-fucosyllactose (3-FL) and/or osteopontin, which may be present together. According to a preferred embodiment the present food products also comprise a combination of 2'-FL and osteopontin. As further supplementary ingredients fat, derived from animal or vegetable sources may be included.

The food composition may be present in liquid or dry form. A liquid food product may be embodied as a beverage, i.e. a liquid infant milk, liquid follow-on or kids milk or a (fruit) juice, or a liquid concentrate. The dry form may be a (base) powder, granules or a tablet.

The food composition envisaged by the present invention may be any of a drinkable or eatable composition, non-limiting examples for which are infant formulas, preferably infant milk formulas in dry or liquid form, adult/elderly bulk dairy powder, food additives, food supplements or also animal feed, e.g. pet food.

According to an embodiment, the food composition is an infant formula, i.e. a formulated milk to be used as a substitute for natural mother's breast milk. In that case, the food composition may also include further nutrients/nutritional components, that are useful or beneficial for infant nutrition, e.g. digestible carbohydrates, preferably oligosaccharides, phospholipids, or milk fat. Also, minerals, or vitamins may be further added substances. The further nutrients, vitamins, minerals may be adjusted to meet recommendations of paediatricians for child's or infant nutrition. The formulation may further be adjusted to meet special nutritional needs of infants or children having a specific allergy or an intolerance or a gastrointestinal disorder. Values for specific nutritional amounts for daily intake may, are known e.g. from a respective national society for nutrition.

According to an embodiment the invention provides the use of the present food compositions containing the same for enhancing and/or supporting a healthy growth and development of a subject. Since the food compositions comprise the whey proteins in an essentially bioactive form as present in milk, among others essential proteins are provided to the individual upon ingestion, which are readily resorbed and provided to metabolic processes, e.g. build-up of muscles, bone strength and quality and joints. The present native whey and the respective compositions are consequently also suitable as an auxiliary measure for muscle build up, e.g. after a disease, under challenging environmental conditions or at an elderly stage of the individual. Likewise for animals, e.g. livestock animal, the weight gain during lifetime may be enhanced.

Likewise the present food compositions may be used for modulating and/or improving the immune status of a subject, or promoting adequate maturation of infant immune system since among others the major native whey proteins as provided herein is known to contain bioactive proteins and immunoglobulins as found in milk, in particular in their active form. Thereby they are susceptible to physiological absorption processes similar to breast milk and generation of smaller protein and peptides, exerting their physiological function in supporting subject's health and immunity.

The present compositions containing native whey proteins are thus useful in assisting subjects to defend themselves from detrimental environmental conditions, such as exposure to viral, bacterial, fungal, parasitic and/or protozoic infections.

Moreover, the present food compositions support intestinal barrier maturation and integrity with the activity of functional native proteins and also a higher concentration of essential amino acids, e.g. lysine, as compared to standard whey, which allows low total protein concentration in infant formula and reduces risk of obesity. The limited presence of glycated proteins produced during a Maillard reaction, which are known to cause intestinal inflammation and allergy, as well supports healthy status of the gastrointestinal system. Overall, native whey proteins and/or food compositions may thus be used in the management or the prevention of a disorder selected from intestinal disorders, metabolic disorders, food allergy as well as an adequate development of the immature infant gastrointestinal tract.

According to another embodiment the present invention discloses a method for producing food compositions containing native whey proteins, essentially maintaining the protein quality and functionality of untreated whey proteins. The method comprises the step of isolating whey proteins from mammalian whole milk or skim milk, in at least one filtration step, mixing the whey proteins thus obtained with other ingredients of a food composition to be prepared and subjecting the mixture to a pasteurisation step at a temperature of from 71.5 to about 82 °C for a time period of from about 10 to about 60 s.

In the present method, according to preferred embodiments the temperature and the duration of exposing the material to the temperature is in a range as described above in connection with the composition.

In order to ensure even distribution of the temperature within the material the whey proteins may be stirred or rearranged in any conventional manner in a suitable container.

After having prepared the native whey proteins containing composition as above the resulting material may by dried, e.g. via spray-drying, or kept in liquid form, or concentrated by evaporation and/or mixed with other food ingredients for further use. Depending on the method of manufacture and final product format, the intended use and/or expected shelf life the resulting product may be stored at ambient or low temperatures, e.g. refrigerator temperatures of from about 4 - 8 °C.

### Definitions

In the context of the present disclosure the following definitions shall generically be applicable.

The terms "whey proteins" are intended to designate mammalian milk proteins essentially comprising albumins and globulins. According to a preferred embodiment said albumins and globulins are selected from among β-lactoglobulin, α-lactalbumin, immunoglobulins, bovine serum albumin, bovine lactoferrin and lactoperoxidase and any other minor proteins in bovine milk. Moreover, "whey" from the milk of other domestic animals with different ratio of proteins is considered as well, including sheep, goat, buffalo and camel.

The term "subject" shall comprise a human or animal at any age. For humans, infants, babies, children, adults, elderly and also older people are envisaged. For animals in particular pets or livestock animals are envisaged such as e.g. dogs, cats, cattle, pig, sheep or goat.

The term "food" or "feed" relates in its widest sense to any composition in dry or liquid form, which is in a form ready-to-ingest or which is embodied as a supplement or additive to other ingestible ingredients.

The invention has now been generically described by way of embodiments, while the disclosure shall explicitly comprise any combination of any of the above described embodiments.

The invention will now be described by way of the following non-limiting examples which are provided for illustrative purposes only.

### Examples

### Example 1: Preparation of Infant Milk Formulas containing whey proteins obtained via micro-filtration and whey proteins obtained from cheese manufacture

Whey protein powder commercially available (containing 80% protein on dry matter) was incorporated in an stage I (0-6 month old) Infant Milk Formula (IMF) composition in an amount of 6.7% together with the other major ingredients such as milk, alpha-lactalbumin, lactose, GOS, vegetable oils, ARA, DHA, vitamins and minerals and mixed using an APV Crepaco high shear mixer. The whey protein powder had been prepared by a process comprising the steps of subjecting skimmed milk to a cascade of microfiltration steps at low temperatures of between 4 - 8 °C and drying the whey proteins at low concentrations to obtain a powder.

The same stage I infant milk formula product was produced using a standard whey protein ingredient obtained from cheese manufacture, by mixing the same amounts of whey proteins and the other ingredients The ingredients and their amounts are listed in table 1.

**Table 1**

| Ingredient (based on dry matter of finished formula) (g/100g) | Standard 1st stage IMF | 1st stage IMF with Native Whey proteins |
|---|---|---|
| Skim Milk | 11 | 11 |
| Standard Whey Protein Ingredient (80% protein on dry matter) | 6,7 | 0 |
| Native Whey Protein Ingredient (80% protein on dry matter) | 0 | 6,7 |
| Alpha-lactalbumin | 1 | 1 |
| GOS | 7,2 | 7,2 |
| Vegetable oil mix | 26 | 26 |
| Soy Lecithin | 0,2 | 0,2 |
| Ascorbyl Palmitate | 0,001 | 0,001 |
| Mixed Tocopherol | 0,013 | 0,013 |
| Arachidonic acid | 0,389 | 0,389 |
| DHA powder | 0,92 | 0,92 |
| Lactose | 43,857 | 43,857 |
| Choline Hydrogen Tartrate | 0,47 | 0,47 |
| Myo-Inositol | 0,035 | 0,035 |
| L-Camitine | 0,016 | 0,016 |
| Taurine | 0,034 | 0,034 |
| Potassium Chloride | 0,1 | 0,1 |
| Sodium Chloride | 0,32 | 0,32 |
| Magnesium chloride | 0,17 | 0,17 |
| Zinc Sulphate | 0,015 | 0,015 |
| Copper Sulphate | 0,0016 | 0,0016 |
| Sodium Selenite | 0,00004 | 0,00004 |
| Magnesium Sulphate | 0,0001 | 0,0001 |
| Potassium Iodide | 0,0002 | 0,0002 |
| Iron pyrophosphate | 0,02 | 0,02 |
| Calcium hydroxide | 0,06 | 0,06 |
| Di potassium Hydrogen Phosphate | 0,6 | 0,6 |
| Tri potassium citrate | 0,01 | 0,01 |
| Calcium Carbonate | 0,39 | 0,39 |
| Nucleotides | 0,028 | 0,028 |
| Vitamin mix | 0,45 | 0,45 |

Wet mix homogenisation was carried out at a pressure of 130/40 bar and the products were pasteurised through a plate heat exchanger (APV) at different temperatures of 75.5°C, 85°C and 95°C for 15 seconds and transferred to an evaporator feed tank to concentrate till a dry matter of 53% was achieved.

The concentrate was further homogenised at 150/50 bar and the fed to a Pilot scale multistage dryer (NIRO) using a high-pressure pump at a flowrate of 180 l/hr.

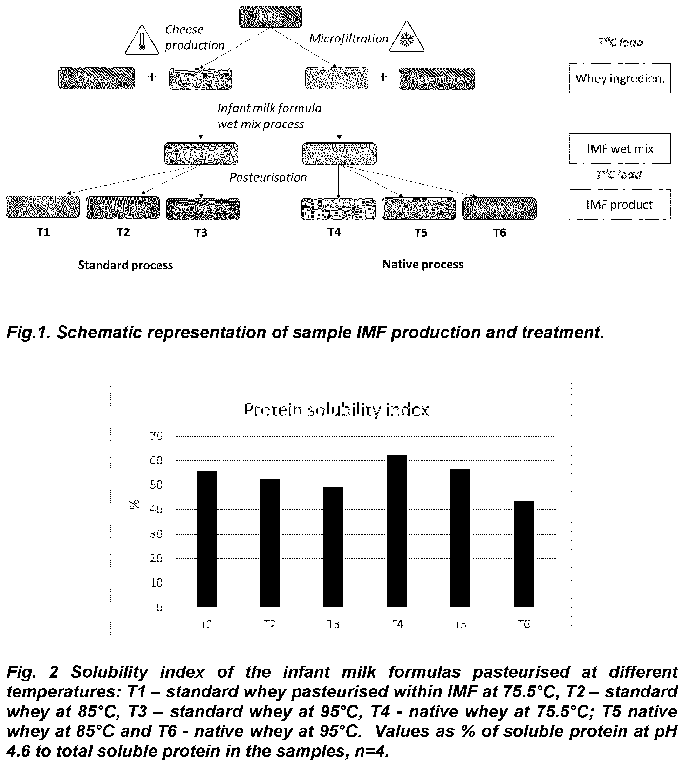

Fig 1 shows schematically the way of producing and treating the different IMF's.

### Example 2: Composition and protein state in IMF containing native whey protein

The compositions were reconstituted as recommended for stage 1 IMFs by dissolving 4.5 g powder in 30 ml water, while stirring and maintaining the mixture at a temperature of 40°C. The pH was measured to be in the range 6.5 - 6.8. The protein solubility index was measured by the Pierce BCA assay as a percentage of protein soluble at pH 4.6 to total protein in the sample. A higher protein solubility index indicates that the protein is in a more native, non-aggregated state and is thereby more available for physiological processes so that it may exhibit its endogenous functionality.

The results are shown in Fig. 2. As is evident therefrom, the sample containing native whey has the highest solubility index clearly indicating that the whey proteins isolated via micro-filtration and included in the IMF essentially preserved their native state.

### Example 3: Determination of IgG and lactoferrin in the IMFs

Concentrations of known functional proteins lactoferrin and IgG were measured in the IMF samples produced in Example 1 by ELISA commercial kits (Cambridge Bioscience). Both lactoferrin and IgG are known to support immunity and resistance to gastrointestinal inflammation in humans, especially at early stages of life.

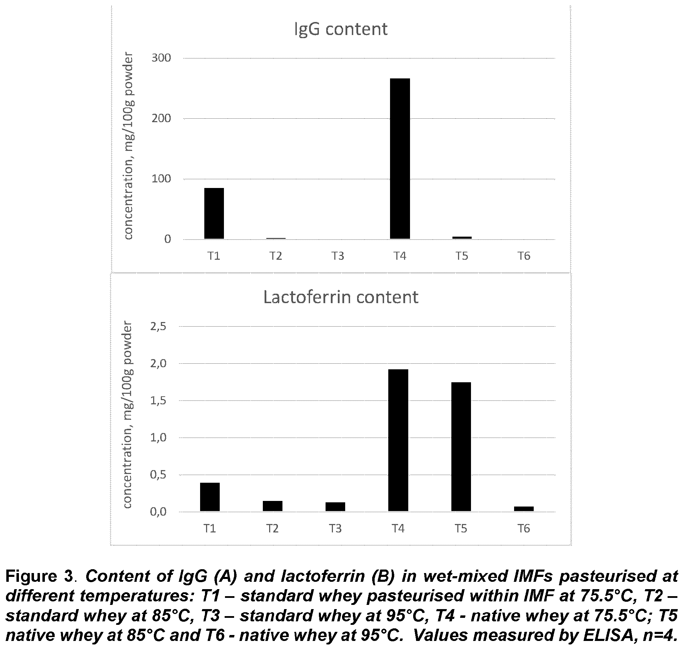

The results are shown in Figure 3 and demonstrate that the highest levels of IgG and lactoferrin were preserved in samples containing native whey and subjected to the pasteurisation temperature (T4). The Lactoferrin content was only slightly reduced when native whey IMF was pasteurised at 85 °C, however the source of whey (cheese whey vs. native whey) had a strong impact.

### Example 4: In-vitro model for Intestinal health and nutrient bioavailability:

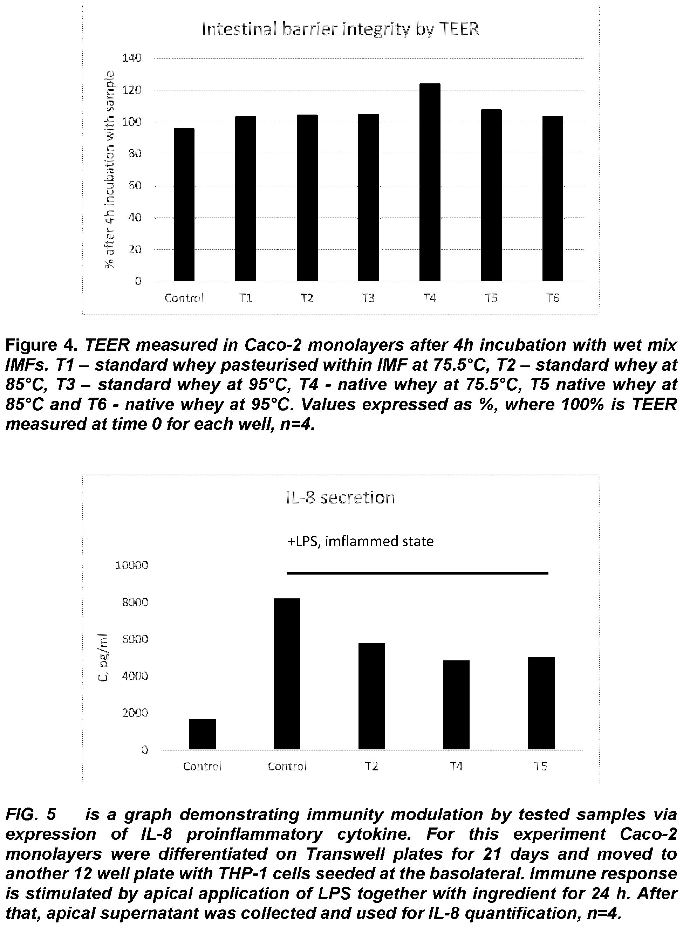

The epithelial colorectal adenocarcinoma cell line, Caco-2, was differentiated in 12-well transwell plates for 20 days and exposed to 125 mM sodium butyrate for 24 h to create "leaky" but healthy phenotype characteristic to infant intestinal barrier, before exposure (No SB - no sodium butyrate "tight" adult intestinal barrier control). The integrity of the monolayer was tested once a week by transepithelial electrical resistance (TEER) using Millicell-ERS. Digested IMFs were prepared as filter-sterilized 5x stocks in HBSS and added at a final protein concentration of approximately 500 µg/cm² to monolayers for 4 h. Apical and basolateral supernatants were collected for bioavailability analysis. TEER measurements were performed to assess model intestinal barrier integrity before and after 4 h treatment (Fig. 4).

### Example 5: +In-vitro model for Immunomodulation

For immunomodulatory analysis THP-1 cells were seeded in 12-well plates and differentiated for 2 -3 days. Differentiated for 21 day Caco-2 monolayers on transwell membranes were added to 12-well plate and exposed to digested and filter-sterilized I60 samples at a final protein concentration of approximately 500 µg/cm² together with immunity response trigger LPS for 24 h. Biomarkers of inflammation/immunity including IL-1β, IL-8, IL-10, and TNFα were quantified in apical cell supernatant with Milliplex Map Kit (HSTCMAG-28SK-06) and MagPix fluorescent detection system according to the manufacturer's instructions. The results for IL-8 are shown in Fig. 5 indicating that the claimed compositions reduced expression of IL-8 most.

## Claims

1. A food composition comprising milk proteins obtained via microfiltration techniques from a milk source and containing whey proteins, which has been treated at a pasteurization temperature of from 71.5 - 82 °C for a time period of from 10-60 s.

2. The food composition according to claim 1, wherein the pasteurization temperature is in a range of from 73.5 - 78°C and the time period is 15-30s.

3. The food composition according to claim 1, comprising the milk proteins obtained via micro-filtration in an amount of from 1 - 80 wt.-%.

4. The food composition according to any of the preceding claims, containing additional ingredients selected from the group comprising excipients, nutrients, minerals, vitamins, oligosaccharides, digestible carbohydrates, amino acids, milk fat, phospholipids and/or any combination thereof.

5. The food composition according to any of the preceding claims, which is present in liquid or dry form.

6. The food composition according to any of the preceding claims, which is an infant formula, nutritional product for children, bulk dairy powder, formulated adult food, a food additive, a food supplement, a pet companion food or an animal feed.

7. The food composition according to claim 6, wherein the infant formula is a stage I formula containing the milk proteins obtained via micro-filtration in an amount of from 6 - 7 wt.-% or stage II or stage III formulas containing native whey proteins in an amount of from 1 - 6 %.

8. Use of a food composition according to any of the preceding claims for enhancing growth and development of a subject and/or intestinal maturation.

9. The food composition according to any of the claims 1 - 7 for use in enhancing the immune status of a subject.

10. The food composition for use according to claim 9 for use in the treatment or prevention of infections and intestinal inflammation.

11. The food composition for use according to any of claims 9 or 10, which is an infant formula, milk and powder beverage for children for catch up growth, being school age and teens - especially in period of high height peak velocity period of between 10- 18yrs.

12. A method for manufacturing a food composition comprising the steps
mixing milk proteins obtained via micro-filtration from a milk source and containing whey proteins with other ingredients of a food composition to be prepared; and
subjecting the mixture to a temperature in the range of from 71.5 - 82 °C for a time period of from 10- 60 s.

13. The method according to claim 12, wherein the temperature is 73.5- 78°C and the time period is 15 -30s.

14. The method according to any of the claims 12 or 11, further comprising the step of spray-drying the resulting food composition containing native whey proteins to obtain a dried powdered product.

15. The method of any of claims 12 - 14, wherein the other food ingredients are selected from excipients, nutrients, minerals, vitamins, oligosaccharides, digestible carbohydrates, amino acids, milk fat, phospholipids and/or any combination thereof.
